# EUROPEAN PATENT SPECIFICATION

(11) **EP 4 069 249 B1**
(45) Date of publication and mention of the grant of the patent: **17.09.2025**
(21) Application number: 20829208.6
(22) Date of filing: 03.12.2020
(51) Int. Cl.: A61K 31/58, A61K 9/14, A61P 27/02, A61P 27/00, A61P 27/14

(54) **COMPOSITION COMPRISING BUDESONIDE FOR OPHTHALMIC USE**
ZUSAMMENSETZUNG MIT BUDESONID ZUR OPHTHALMISCHEN VERWENDUNG
COMPOSITION COMPRENANT DU BUDÉSONIDE POUR USAGE OPHTALMIQUE

(30) Priority: 03.12.2019 IT 201900022860; 03.12.2019 IT 201900022881
(43) Date of publication of application: 12.10.2022
(73) Proprietor: NTC S.r.l., 20124 Milano (IT)
(72) Inventor: MARCELLONI, Luciano, 20124 Milano (MI) (IT); BERTOCCHI, Federico, 20124 Milano (MI) (IT)
(74) Representative: Benedetto, Marco
(86) International application number: PCT/IB2020/061444
(87) International publication number: WO 2021/111358

(56) References cited:
- CN-A- 101 987 101
- US-A1- 2009 312 724
- DATABASE WPI Week 201616, Derwent World Patents Index; AN 2016-124298, XP002799792

## Description

### FIELD OF THE INVENTION

The present invention relates to budesonide, or a salt thereof, or mixtures thereof for ophthalmic use in a method for the curative treatment of an eye disorder or ailment or disease in a subject in need, preferably wherein said method comprises an administration of budesonide to said subject through the ocular topical route, as defined in the appended claims. Furthermore, the present invention relates to a composition comprising a mixture comprising or, alternatively, consisting of: (i) a budesonide, or a salt thereof, or mixtures thereof; (ii) at least one suspending/thickening agent, and optionally (iii) one or more physiologically and/or pharmacologically acceptable excipients; said composition being for use in a method for the curative treatment of an inflammation of the ocular adnexa or inflammation of the eyeball in a subject in need, as defined in the appended claims.

Furthermore, disclosed in the present description is a method for the curative treatment of an inflammation of the ocular adnexa or inflammation of the eyeball in a subject in need, wherein said treatment method comprises administering said composition to said subject.

### BACKGROUND OF THE INVENTION

The ocular adnexa include the ocular muscles, the eyelids, the lacrimal apparatus. Numerous anatomica structures provide the eye with protection, lubrication of the surface exposed to the external, possibility of movement. At the front, the eyelids constitute curtains, which, if necessary, close the orbital cavity. The lacrimal apparatus, in its secretion portion (main and accessory lacrimal glands), and outflow (canals, lacrimal sac, nose-lacrimal duct), provides for the lubrication and hydration of exposed surfaces and the removal of dirt. The conjunctiva forms sacs that allow ocular movements, protecting the sclera and closing the orbital cavity. The 6 extrinsic eye muscles, inserted on the orbital walls and on the sclera, move the eye. The eye, or eyeball, is the main sense organ of the visual apparatus, which has the task of obtaining information about the surrounding environment through light. The conjunctiva is the membrane that internally covers the eyelid and folds to cover the sclera (the robust white fibrous membrane that covers the eye) up to the edge of the cornea (the transparent layer in front of iris and pupil). The conjunctiva protects the eye by keeping foreign bodies and microorganisms that cause infections away and contributing to the maintenance of the tear film. The most frequent condition of the conjunctiva is inflammation (conjunctivitis). There are many causes of inflammation, including bacterial infections (including chlamydia), viruses or fungi; allergic reactions; chemical substances or foreign bodies in the eye; and excessive exposure to sunlight. The cornea is the transparent part of the eye found in front of the pupil and the iris (the coloured part of the eye).

Today, numerous pharmaceutical products are available for the treatment of inflammatory diseases of the eye, useful to reduce inflammation that may affect the ocular adnexa or the eyeball. Said pharmaceutical products for the treatment of inflammatory diseases contain non-steroidal anti-inflammatory active substances (NSAIDs; *non-steroidal anti-inflammatory drugs*), or steroidal (corticosteroids).

Non-steroidal active substances act on the metabolism of arachidonic acid and eicosapentaenoic acid, precursors of molecules involved in the inflammatory process such as prostaglandins (PG), prostacyclins (PC), thromboxanes (TX) and leukotrienes (LT).

Steroidal active substances possess a sterol nucleus (cyclopentanoperhydrophenanthrene) consisting of four fused rings - three six-atom rings (A, B, C) and a five-atom ring (D) - as represented in the following general formula (I):

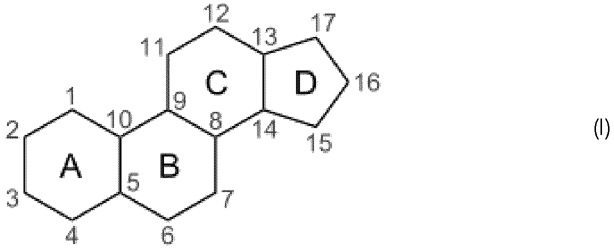

The molecule called budesonide (CAS No. 51333-22-3) is a steroidal anti-inflammatory agent used locally in some topical regions, being used in particular through the inhalation route in the prevention of asthma attacks (in the form of a suspension by nebulisation for inhalations or nasal spray, or in the form of dry powder for inhalation), or through the enteral route in the treatment of Crohn's disease (in the form of a coated capsule). When budesonide reaches the liver carried by the bloodstream, budesonide is metabolised to pharmacologically inactive by-products. For this reason, budesonide is used only locally.

Budesonide has peculiarities of action that make it an active ingredient with an "inherently delayed" action. In (Magnus JENDBRO et al. "Pharmacokinetics of budesonide and its major ester metabolite after inhalation and travenous administration of budesonide in the rat"; Drug Metabolism and Disposition, The American Society of Pharmacology and Experimental Therapeutics, Vol. 29, No. 5 (1998); Elisabet WIESLANDER et al. "Pharmacologic Importance of the Reversible Fatty Acid Anjugation of Budesonide Studied in a Rat Cell Line In Vitro"; American Journal of Respiratory Cell and Molecular Biology; Vol 19 (1998), 477-484") it has been shown that such delayed action is due to a longer action time with respect to other corticosteroids (for example loteprednol or dexamethasone), necessary for reversible intracellular esterification of budesonide to occur, in an esterified form and in a non-esterified form.

Budesonide has the following general formula (II):

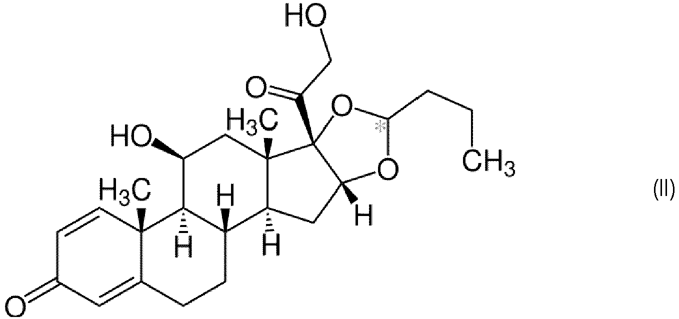

Budesonide is not water-soluble. The pharmaceutical formulations based on budesonide, present on the market, therefore provide for the presence of said steroidal anti-inflammatory agent in solid form (for example as powder, granulate or tablets), or in aqueous suspension.

Budesonide aqueous suspension formulations are recommended for the local symptomatic treatment of allergic rhinitis. Such symptoms comprise swelling of the mucous membranes due to an inflammation and phlogosis originating from pollen, dust, mites or other foreign antigenic materials. Said aqueous suspension formulations used in the nasal region help to successfully limit the inflammations induced by allergic reactions.

The suspension budesonide formulations theoretically further prolong the inherently delayed action of budesonide, by virtue of a slow process of dissolution of such corticosteroid, which is therefore not immediately available for absorption since they are not dissolved in the biological fluid outside the tissue. US 2009/312724 A1 discloses an unscented metered-dose manual-pump spray formulation (for nasal administration) containing a suspension of micronized budesonide in an aqueous medium (product Rhinocort Aqua^{®}). Other components of the formulation are: (1) a mixture of microcrystalline cellulose and sodium carboxymethyl cellulose, (2) dextrose, (3) polysorbate 80, (4) disodium edetate and (5) potassium sorbate. US 2009/312724 A1 does not disclose the use of the product Rhinocort Aqua^{®} in a method for the curative treatment of an inflammation of the ocular adnexa or an inflammation of the eyeball in a subject in need.

There are several eye drops based on non-steroidal anti-inflammatories (NSAIDs) on the market. The main drawbacks of NSAID-based eye drops are conjunctival redness and burning, eyelid oedema and anterior segment structures of the eye, keratitis and itching. Steroid eye drops containing loteprednol (Lotemax^{®}) or dexamethasone (Maxidex^{®}) also have side effects including: feeling of discomfort in the eye, feeling of foreign body in the eye, corneal defects, ocular itching, increased lacrimation, irritation, redness.

The prior document CN 101 987 101 A relates to an anti-inflammatory ophthalmic composition comprising a glucocorticoid, preferably budesonide, cited in a first list comprising tens of possible alternatives. Said composition is preferably eye drops in the form of an ophthalmic solution or suspension, preferably an ophthalmic ointment or an ophthalmic gel. The ophthalmic gel comprises one or more from a buffer agent, an isotonic agent, an antibacterial agent, a stabiliser agent, an antioxidant, a surfactant, an acidity-basicity regulator, a chelating agent, an absorption enhancer, a thickening agent, a wetting agent (second list of components). A stabiliser agent for said gel is carboxymethylcellulose, selected from a third list comprising a series of possible alternatives.

None of the compositions discussed in the examples of prior document CN 101 987 101 A contains budesonide, nor only the soluble fractions of carboxymethylcellulose. Furthermore, in such examples, budesonide in the form of micronized powder is not present.

Prior document US 2009/312724 A1 discloses aqueous solution corticosteroid formulations for use in the treatment of nasal and/or ophthalmic diseases or symptoms. In an embodiment the corticosteroid is budesonide. Paragraph [0152] of prior document US 2009/312724 A1 specifies that, in order to maximise nasal administration, said formulation may have a mass median aerodynamic diameter (MMAD) of at least 3.5 µm, of at least 5 µm, of at least 10 µm, of at least 20 µm or of at least 35 µm. Paragraph [0179] of such document discloses commercial formulations of budesonide. Among these, PULMICORT RESPULES suspension, is a sterile aqueous suspension of micronized budesonide which can be administered by inhalation by means of a nebuliser.

None of the formulations discussed in the examples of prior document US 2009/312724 A1 contains only the soluble fractions of sodium carboxymethylcellulose. Furthermore, none of the formulations shown in such document uses micronized budesonide for use in a method for the curative treatment of an inflammation of the ocular adnexa or inflammation of the eyeball.

The need is therefore felt to be able to have a composition capable of allowing to obtain an anti-inflammatory action that is effective and prolonged over time so as to avoid multiple administrations which unnecessarily increase the feeling of burning and itching. Furthermore, the need is felt to be able to have a composition which is not affected by the existing problems and limits of the currently existing formulations, and which is tolerated by a greater number of subjects.

After a long and intense research and development activity, the Applicant developed an ophthalmic composition capable of providing an adequate response to the limits, drawbacks and problems currently existing in products on the market.

### DETAILED DESCRIPTION

Forming an object of the present invention is budesonide, or a salt thereof, or mixtures thereof for ophthalmic use in a method for the curative treatment of an eye disorder or ailment or disease in a subject in need, preferably wherein said method comprises an administration of said budesonide to said subject through the ocular topical route, having the characteristics as defined in the attached claims.

In particular, forming an object of the present invention is a composition comprising a mixture comprising, or alternatively, consisting of:
(i) budesonide, or a salt thereof, or mixtures thereof, in form of micronized powder, wherein said micronized powder comprises powder particles having an average size distribution comprised from 0.05 µm to 20 µm;
(ii) at least one suspending/thickening agent selected from the group comprising or, alternatively, consisting of: carboxymethylcellulose (CMC), sodium carboxymethylcellulose (Na-CMC) and refined carboxymethylcelluloses, or mixtures thereof, wherein said composition comprises only the water-soluble fractions of CMC, Na-CMC and/or refined carboxymethylcelluloses, wherein said water-soluble fractions of carboxymethylcellulose (CMC), sodium carboxymethylcellulose (Na-CMC) and/or refined carboxymethylcelluloses exclude products containing or, alternatively, consisting of microcrystalline cellulose and sodium carboxymethylcellulose at variable ratios and having non-water-soluble fractions, and optionally
(iii) one or more physiologically and/or pharmacologically acceptable excipient,
wherein said composition is for use in a method for the curative treatment of an inflammation of the ocular adnexa or an inflammation of the eyeball in a subject in need.

Furthermore, disclosed in the present description is a method for the curative treatment of inflammation of the ocular adnexa or an inflammation of the eyeball in a subject in need, wherein said treatment method comprises administering said composition to said subject, having the characteristics as defined in the attached claims.

Preferred embodiments of the present invention will be described hereinafter by way of non-limiting example with the aid of the attached drawings, wherein some indices of the eye inflammatory condition are measured:
- Figure 1 shows - in graphic form - the results of the first observations of Example 2 relating to the possible material suspended in water after 2 hours;
- Figure 2 shows - in graphic form the results of the second observations of Example 2 relating to the protein concentration (as an index of inflammation) after 2 hours;
- Figures 3 and 4 respectively show a diagram of the Cumulative Distribution Q3% (HELOS Particle Size Analysis) with respect to the particle size in µm, and a table corresponding to said diagram of the Cumulative Distribution with standard deviation, according to a possible embodiment of the budesonide that can be used in this invention. Figure 4 shows that x0/µm = 2.50 has a Q3% equal to 90.32 and that x0/ µm =3.75 has a Q3% equal to 99.01. In this range of there is half the total weight, almost as in the remaining 100%;
- Figure 5 discloses a diagram of the average size distribution of a budesonide in the form of micronized powder, obtained by means of a laser diffractometer, according to another embodiment of the present invention.

The present invention relates to budesonide, or a salt thereof, or mixtures thereof for ophthalmic use in a method for the curative treatment of an eye disorder or ailment or disease in a subject in need, preferably wherein said method comprises an administration of said budesonide to said subject through the ocular topical route.

The disorder or said ailment or said disease is an inflammation of the ocular adnexa or an inflammation of the eyeball that can be of various origins: microbiological (virus and pathogens), mechanical or particle, or allergic.

Inflammation of the ocular adnexa is preferably selected from the group comprising or, alternatively, consisting of conjunctivitis, blepharitis, blepharoconjunctivitis, chalazion, dacryocystitis.

Inflammation of the eyeball is preferably selected from the group comprising or, alternatively, consisting of keratitis, keratoconjunctivitis, scleritis or episcleritis, uveitis.

More preferably, said disorder or said ailment or said disease is an eye inflammation selected from the group comprising, or alternatively, consisting of allergic conjunctivitis, allergic keratitis, keratitis, dry eye syndrome, allergy eye redness, and swelling from ocular phlogosis.

However, in the context of the present invention we consider that said budesonide, or salt thereof, or mixtures thereof, is used and administered in the form of an aqueous suspension composition given that said budesonide is in the form of a powder - in short, a powder in micronized form. Said budesonide in the form of a powder contains a micronized powder with an average size comprised between 0.05 µm and 20 µm which, in turn, contains a fraction of an ultrafine micronized powder having an average size distribution less than or equal to 10 µm (preferably comprised from 0.1 µm to 10 µm).

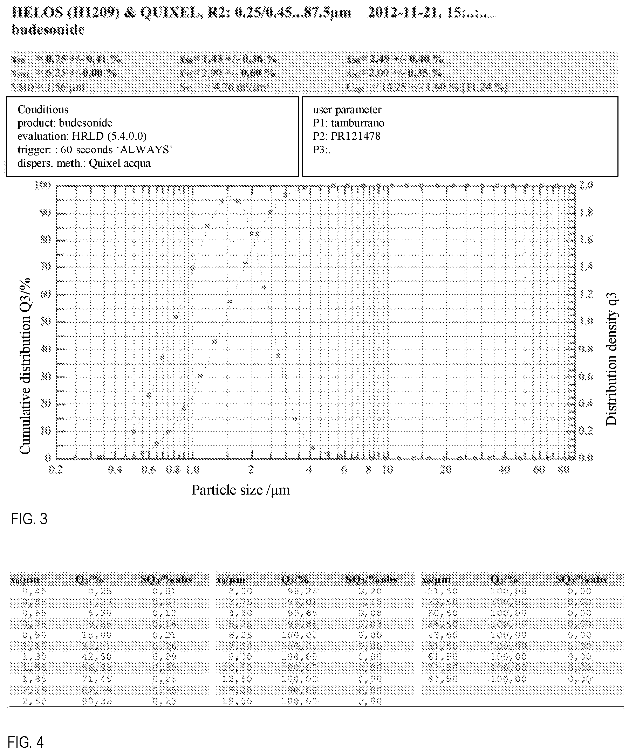

Said budesonide is obtained and supplied by the manufacturer already in this form according to an average size distribution shown in the diagram of Figure 3, and in tabular form in Figure 4, which show the cumulative distribution Q3% with respect to the particle size in µm.

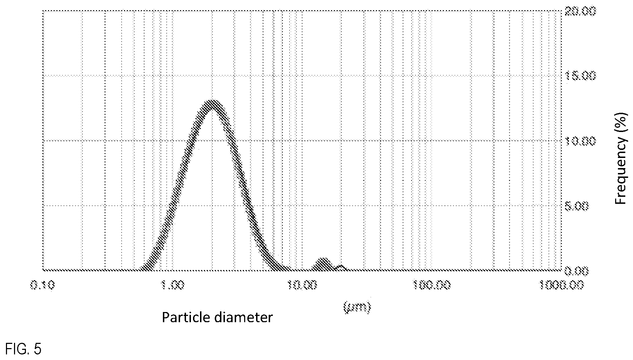

Preferably, said budesonide has an average size distribution shown in the diagram of Figure 5, and in tabular form in Table A.1 below (in which the numerical values are expressed in µm).

**Table A.1**

| | | |
|---|---|---|
| **Dx(10)** | **Dx(50)** | **Dx(90)** |
| 1.10 | 2.02 | 3.68 |

In Table A.1, the parameters Dx(10), Dx(50) and Dx(90) respectively indicate that 10%, 50% and 90% by weight of the particles analysed have a dimensional distribution below the numerical values (expressed in µm) indicated in such Table.

Said powder in the micronized form of budesonide preferably comprises - on average - substantially spherical-shaped powder particles.

In this description, the expression "micronized" means that substantially the entire particle population of said powder comprises particles having an average size comprised from 0.05 µm to 20 µm, more preferably comprised from 0.1 µm to 10 µm, even more preferably comprised from 0.2 µm to 5 µm, even more preferably comprised from 0.25 µm to 3.75 µm, even more preferably comprised from 0.40 µm to 3.0 µm.

In this description, "substantially the entire particle population" means that a total percentage by volume equal to or greater than 90%, i.e. comprised from 94% to 100%, preferably from 94% to 98%, of said powder particles has a distribution comprised from 0.05 µm to 20 µm, more preferably comprised from 0.1 µm to 10 µm, even more preferably comprised from 0.2 µm to 5 µm, even further more preferably comprised from 0.25 µm to 3.75 µm, most preferably comprised from 0.40 µm to 3.0 µm.

By way of example, such average size distribution is determined according to the calculation of the mean geometric diameter for number determined with the aid of modern electronic equipment such as light scattering or laser. The same results can be obtained through visual observation and field count under the microscope.

For example, the average size distribution could be determined by means of a Spraytec laser diffractometer (Malvern Panalytics, UK), preferably using the instrumental operating parameters shown in Table A.2 below, where the parameters not specified in such table were maintained as per default specifications of the laser diffractometer.

**Table A.2**

| ***Parameter*** | ***Value*** |
|---|---|
| Optical properties of the dispersing agent | Cvclohexane (refraction index 1.43) |
| Focal Lens | 300 mm* |
| Detector Range | 10 - last |
| Type of measurement | Time-based |
| Time of measurement | 1 minute, sampling each second |

| | |
|---|---|
| *The lens allows to measure particles in the range comprised from 0.1 µm - 900 µm (Dv₅₀ 0.5 µm - 600 µm). | |

In the presence of asymmetric powder particles (with non-spherical symmetry), the so-called *"equivalent spherical diameter*", which compares the particle size with the diameter of a sphere having the same volume, the same area, the same projection area and the same volume/surface ratio, is preferably used. The equivalent spherical diameter corrects or recalculates the size of a non-spherical particle according to the diameter of a sphere having the same volume.

Said average size distribution less than or equal to 20 µm (preferably comprised from 0.05 µm to 20 µm) does not show contact irritant effects, and it is sufficiently fine to avoid the perception of powder particles by the eye as a solid.

Said powder in micronized form of budesonide is preferably suspended in a liquid carrier, preferably an aqueous carrier, more preferably water, even more preferably purified water or water for injections.

The average particle size distribution of the powder particles comprised from 0.05 µm to 20 µm, preferably comprised from 0.1 µm to 10 µm, more preferably comprised from 0.2 µm to 5 µm, even more preferably comprised from 0.25 µm to 3.75 µm, more preferably comprised from 0.40 µm to 3.0 µm, relates to the only material in suspension detectable within said composition, which is preferably only budesonide. As a matter of fact, said composition is preferably devoid of opaque ingredients.

In the present description, the term *"opaque ingredients"* is used to indicate additional ingredients with respect to budesonide, or salt thereof, or mixtures thereof which, suspended in said composition, could render said composition less transparent to visible light with respect to a composition devoid of said ingredients. The opaque ingredients are thus ingredients suspended and undissolved in said composition, and additionally with respect to budesonide, or salt thereof, or mixtures thereof.

In other words, said composition comprises, additionally to (i), (ii) and one or more optional (iii) physiologically and/or pharmacologically acceptable excipients dissolved in solution which, since solubilised, do not have an opacifying effect on the composition and they do not generate contact irritant effects.

In the context of the present invention, the term "composition/s" is used to indicate a pharmaceutical composition, or a medical device composition.

In an embodiment, said composition is a liquid composition, preferably in the form of a suspension in water, more preferably purified water or water for injections. More preferably, said suspension has a pH value comprised from 6±0.1 to 8±0.1, preferably comprised from 6.5±0.1 to 7.5±0.1, even more preferably from 6.7±0.1 to 7.3±0.1 (at 20°C and 1 Atm).

Said method comprises an administration of said composition through the ocular topical route, preferably on the ocular adnexa or on the eyeball, more preferably on the conjunctiva and cornea, even more preferably on the cornea (with eye open).

An amount of said (i) budesonide, or salt thereof, or mixtures thereof is comprised from 0.005% (w/v) to 3% (w/v) by weight, with respect to a total volume of said liquid composition; preferably comprised from 0.01% (w/v) to 2% (w/v) by weight; even more preferably comprised from 0.05% (w/v) to 1.5% (w/v) by weight; for example 0.1% (w/v), 0.2% (w/v), 0.3% (w/v), 0.4% (w/v), 0.5% (w/v), 0.6% (w/v), 0.7% (w/v), 0.8% (w/v), 0.9% (w/v) or 1% (w/v). Even more preferably, the amount of said (i) budesonide, or salt thereof, or mixtures thereof in said liquid composition is comprised from 0.05% (w/v) to 0.2% (w/v), preferably comprised from 0.07% (w/v) to 0.15% (w/v), even more preferably comprised from 0.08% (w/v) to 0.12% (w/v).

By way of example, an amount from 0.05 g to 1.5 g, for example 0.1 g, 0.2 g, 0.3 g, 0.4 g, 0.5 g, 0.6 g, 0.7 g, 0.8 g, 0.9 g or 1.0 g of said budesonide (i), or salt thereof, or mixtures thereof could be suspended in 100 ml of the liquid of said liquid composition.

The use of a low dose /concentration of budesonide powder in micronized form in the composition subject of the present invention (containing or consisting of a micronized powder with an average size distribution comprised from 0.05 µm to 20 µm and/or an ultrafine micronized powder with an average size distribution less than or equal to 10 µm (preferably comprised between 0.1 µm and 10 µm)) comprised from 0.01% (w/v) to 0.3% (w/v) by weight, preferably comprised from 0.05% (w/v) to 0.2% (w/v) by weight, even more preferably comprised from 0.08% (w/v) to 0.12% (w/v), for example 0.1% (w/v) by weight, allows to have a smaller micro-particle fraction in suspension and, thus, budesonide can be suspended more effectively by the suspending/thickening agent. Thus, a smaller amount of suspending/thickening agent necessary to suspend a low dose of budesonide powder in micronized form, comprised from those mentioned above, makes a drop of composition of the present invention in the form of a suspension being applied less viscous (preferably a viscosity comprised from 10 CPS to 25 CPS measured at 20°C and 1 Atmosphere of ambient pressure, more preferably comprised from 15 CPS to 25 CPS, for example 11 CPS, 13 CPS, 15 CPS, 17 CPS, or 19 CPS) and more spreadable.

In the composition of the present invention, said (ii) at least one suspending/thickening agent used in combination with the (i) budesonide, or a salt thereof, or mixtures thereof, is selected from the group comprising or, alternatively, consisting of: carboxymethylcellulose (CMC), sodium carboxymethylcellulose (Na-CMC), refined carboxymethylcelluloses, or mixtures thereof, preferably in cross-linked form/s.

Said suspending/thickening agent is present in said mixture at an amount by weight comprised from 0.005% (w/v) to 3% (w/v) by weight, with respect to a total volume of said composition; preferably at an amount by weight comprised from 0.01% (w/v) to 2% (w/v) by weight; more preferably comprised from 0.05% (w/v) to 1.5% (w/v) by weight; even more preferably comprised from 0.1% (w/v) to 1% (w/v); for example 0.12% (w/v), 0.14% (w/v), 0.16% (w/v), 0.18% (w/v), 0.20% (w/v), 0.25% (w/v), 0.30% (w/v), 0.4% (w/v), 0.5% (w/v), 0.6% (w/v), 0.7% (w/v), 0.8% (w/v), 0.9% (w/v), or 1% (w/v).

The (ii) suspending/thickening agent, used in combination with the component (i) of the composition of the present invention, is selected from cellulose polymers having only the water-soluble fractions of carboxymethylcellulose (CMC), sodium carboxymethylcellulose (Na-CMC) and/or refined carboxymethylcelluloses. These cellulose polymers are transparent (i.e., non-opaque) when dissolved in the liquid carrier, for example water, since they do not contain solid residues and they can be fully gelled in water without suspended particles with an average size distribution of component (i) greater than 20 µm being irritating to the eye.

The cellulose polymers having only the water-soluble fractions of carboxymethylcellulose (CMC), sodium carboxymethylcellulose (Na-CMC) and/or refined carboxymethylcelluloses have a minimum carboxymethylcellulose content comprised from 97% to 100% by weight, preferably from 98% to 99.9% by weight, in water at 25°C and 1 atmosphere pressure. Said water-soluble fractions of CMC, Na-CMC and/or refined carboxymethylcelluloses preferably have an amount of sodium comprised from 7.0% to 9.5% by weight, more preferably comprised from 7.5% to 9.3% by weight, even more preferably comprised from 8.2% to 9% by weight. Said water-soluble fractions of carboxymethylcellulose (CMC), sodium carboxymethylcellulose (Na-CMC) and/or refined carboxymethylcelluloses carboxymethylcellulose have a bulk density comprised from 0.45 g/cm³ to 1.05 g/cm³, preferably comprised from 0.55 g/cm³ to 1.00 g/cm³ (measured according to known techniques and equipment).

Said at least one agent (ii) suspending/thickening agent, used in combination with the component (i) in the liquid composition of the present invention, is - as mentioned - selected from cellulose polymers having only the water-soluble fractions of carboxymethylcellulose (CMC), sodium carboxymethylcellulose (Na-CMC) and/or refined carboxymethylcellulose. These cellulose polymers have the advantage of controlling and delaying the delivery of (i) budesonide, or salt thereof, or mixtures thereof at the interface with cellular tissues, since only the water-soluble fractions of refined CMC, Na-CMC and/or refined carboxymethylcelluloses act as a water-soluble matrix for budesonide.

Such delivery delay is particularly marked when budesonide is in form of micronized powder comprising powder particles having an average size distribution comprised from 0.05 µm to 20 µm, preferably comprised from 0.1 µm to 10 µm, more preferably comprised from 0.2 µm to 5 µm, even more preferably comprised from 0.25 µm to 3.75 µm, more preferably comprised from 0.40 µm to 3.0 µm.

Cellulose polymers having only the water-soluble fractions of carboxymethylcellulose (CMC), sodium carboxymethylcellulose (Na-CMC) and/or refined carboxymethylcelluloses exclude products containing or, alternatively, consisting of microcrystalline cellulose and sodium carboxymethylcellulose at variable ratios and having non-water-soluble fractions. Such products are known under the trade names Avicel^{®} RC581, Avicel^{®} RC591 and Avicel^{®} CL611. By way of example, the safety data sheet of the product Avicel^{®} CL611 indicates, with respect to solubility in water, that such product may be dispersed (but not dissolved) in water.

Refined carboxymethylcelluloses (for example that manufactured by Blanose^{™}) is a purified salt of sodium carboxymethylcellulose (Na-CMC) with a minimum carboxymethylcellulose content (CMC) content comprised from 95% to 99.9%, preferably comprised from 96% to 98%. Refined Na-CMC is a water-soluble anionic polymer which acts as suspending/thickening agent in the composition of the present invention.

Refined Na-CMC may have two degrees of substitution (DS) and some degrees of viscosity.

The degree of substitution (DS) is defined as the average number of hydroxyl groups substituted with anhydroglucose units (AGU) in said refined CMC.

Typical substitution levels and viscosity ranges are indicated below. Generally, higher levels of substitution enhance compatibility with other components of the composition/system. More precisely, higher substitution levels enhance the water solubility of the refined Na-CMC.

**Table 1**

| **Degree of substitution. Method: MA 304.1506°** | | |
|---|---|---|
| **Type of DS** | **Limits of substitution** | **Sodium content** |
| **7 (^{a})** | From 0.65 to 0.90 | 7.0 - 8.9 |
| | From 0.80 to 0.95 | 8.2 - 9.3 |

| | | |
|---|---|---|
| (^{a}) some degrees have a narrower substitution range | | |

Therefore, the refined CMC in said composition preferably has a substitution limit comprised from 0.65 to 0.90 or from 0.80 to 0.95, more preferably comprised from 0.80 to 0.95.

**Table 2**

| **Typical viscosity ranges. Method MA 304.1001A** | | | | | |
|---|---|---|---|---|---|
| DS7 Degrees | DS9 Degrees | Concentrations | Range 25° C mPa-s | Brookfield LVF settings | |
| | | | | Ago n. | tpm |
| 7H9 | | 1 | 4,000 - 9,000 | 4 | 30 |
| 7H4 | 9H4 | 1 | 2,500 - 4,500 | 4 | 30 |
| 7H | | 1 | 1,500 - 2,500 | 3 | 30 |
| 7M65 | | 2 | 3,000 -6,500 | 4 | 30 |
| 7M31 | | 2 | 1,500 -3,100 | 3 | 30 |
| 7M | | 2 | 300 -600 | 2 | 30 |
| 7M2 | 9M2 | 2 | 100 -200 | 2 | 60 |
| 7M1 | | 2 | 50 -100 | 1 | 60 |
| 7L | | 2 | 25 -50 | 1 | 60 |
| 7L2 | | 6 | 120 -280 | 2 | 60 |
| 7L1 | | 6 | 90 -130 | 2 | 60 |
| 7EL | | 6 | 35 -60 | 1 | 60 |
| 7UL | | 6 | 10 -25 | 1 | 60 |

| | | | | | |
|---|---|---|---|---|---|
| This table indicates the available viscosity levels. | | | | | |

### Other typical properties of refined CMC (Table 3):

**Table 3**

| | | |
|---|---|---|
| **Properties** | **Limits** | **Method** |
| Purity, 100 - NaCI + Na glycolate), % | 98 min. | MA 304.1501A/1502A |
| Bulk density, g/cm³ | 0.55 - 1.00 | MA 304.1607A |
| Humidity at packaging, % | 8 max. | MA 304.1600A |
| pH of the solutions, | | MA 304.1004A |
| All types L, at 2% | 5.0 - 8.5 | |
| All types M, at 1% | 6.5 - 8.5 | |
| All types M, at 1% | 6.5 - 8.5 | |

### Size gradings of refined CMC. Method 304.1602A (Table 4):

**Table 4**

| **Designation** | **Description** | **Limitation** | | **Opening of the sieve** | |
|---|---|---|---|---|---|
| | | | | **mm** | **ASTM n.** |
| X | Thin | 0.5% max. | MAINTAINED | 0.250 | 60 |
| | | 80% mon. | PAST | 0.075 | 200 |
| - | Normal | 1% max. | MAINTAINED | 0.600 | 30 |
| | | 10% max. | MAINTAINED | 0.425 | 40 |
| C | Granulate | 0% | MAINTAINED | 1.70 | 12 |
| | | 50% max. | MAINTAINED | 0.85 | 20 |
| | | 50% max. | PAST | 0.425 | 40 |
| | | 15% max. | PAST | 0.18 | 80 |
| C1 | Granulate | 0% | MAINTAINED | 1.70 | 12 |
| | | 50% max. | MAINTAINED | 0.85 | 20 |
| | | 50% max. | PAST | 0.18 | 80 |
| C2 | Granulate | 0% | MAINTAINED | 1.70 | 12 |
| | | 10% max. | PAST | 0.18 | 80 |

The Na-CMC used in said composition preferably has CAS No. 9004-32-4 and it is devoid of microcrystalline cellulose (CAS No. 9004-34-6).

Besides (i) and (ii), the composition of the present invention further comprises or consists of optional (iii) physiologically and/or pharmacologically acceptable excipients which may preferably contribute to formulating the composition in the form of an aqueous suspension for topical use, for example as eye drops.

The physiologically and/or pharmacologically acceptable excipients are selected from the group comprising or, alternatively, consisting of: a preservative, preferably potassium sorbate and/or benzalkonium chloride, a surfactant, preferably polyoxyethylene (20) sorbitan monooleate (Polysorbate 80), a chelating agent, preferably ethylenediaminetetraacetic acid (EDTA), or a salt thereof, or mixtures thereof, an isotonic agent, preferably mannitol or sodium chloride, a carrier, preferably an aqueous carrier, more preferably water, or mixtures thereof.

In an embodiment, the composition of the present invention may comprise:
(i) budesonide, or a salt thereof, or mixtures thereof, as defined in appended claim 1;
(ii) at least one suspending/thickening agent selected from carboxymethylcellulose (CMC), sodium carboxymethylcellulose (Na-CMC), refined carboxymethylcellulose, or mixtures thereof, as defined in appended claim 1;
(iii.a) potassium sorbate (preservative);
(iii.b) polyoxymethylene (20) sorbitan monooleate (Polysorbate 80) (surfactant);
(iii.c) ethylenediaminetetraacetic acid (EDTA), or a salt thereof, or mixtures thereof (sequestrant);
(iii.d) mannitol or sodium chloride (tonicity agent);
(iii.e) benzalkonium chloride (preservative);
(iii.f) water, preferably purified water or water for injections (liquid carrier).

In an embodiment, the composition of the present invention may comprise the following amounts expressed as weight with respect to total volume of said composition (w/v):
(i) budesonide, or a salt thereof, or mixtures thereof at an amount comprised from 0.01% (w/v) to 0.3% (w/v) by weight, preferably comprised from 0.07% (w/v) to 0.2% (w/v) by weight, even more preferably comprised from 0.08% (w/v) to 0.15% (w/v), for example 0.1% (w/v) by weight;
(ii) carboxymethylcellulose (CMC), sodium carboxymethylcellulose (Na-CMC), refinec carboxymethylcellulose, or mixtures thereof, at an amount comprised from 0.05% (w/v) to 1.5% (w/v) by weight; preferably comprised from 0.1% (w/v) to 1% (w/v); for example 0.12% (w/v), 0.14% (w/v), 0.16% (w/v), 0.18% (w/v), 0.20% (w/v), 0.25% (w/v), 0.30% (w/v), 0.4% (w/v), 0.5% (w/v), 0.6% (w/v), 0.7% (w/v), 0.8% (w/v), 0.9% (w/v), or 1% (w/v);
(iii.a) potassium sorbate at an amount comprised from 0.005% (w/v) to 3% (w/v), preferably comprised from 0.01% (w/v) to 2% (w/v), more preferably comprised from 0.05% (w/v) to 1.5% (w/v), for example 0.100% (w/v);
(iii.b) polyoxymethylene (20) sorbitan monooleate (Polysorbate 80) at an amount comprised from 0.001% (w/v) to 2% (w/v), preferably comprised from 0.005% (w/v) to 1% (w/v), even more preferably comprised from 0.01% (w/v) to 1.5% (w/v), for example 0.030% (w/v);
(iii.c) ethylenediaminetetraacetic acid (EDTA), or a salt thereof, or mixtures thereof at an amount comprised from 0.005% (w/v) to 3% (w/v), preferably comprised from 0.01% (w/v) a 2% (w/v), more preferably comprised from 0.05% (w/v) to 1.5% (w/v), for example 0.100% (w/v);
(iii.d) mannitol or sodium chloride, preferably mannitol, at an amount comprised from 0.01% (w/v) to 10% (w/v), preferably comprised from 0.1% (w/v) to 7% (w/v), more preferably comprised from 0.5% (w/v) to 5% (w/v), even more preferably comprised from 0.8% (w/v) to 3% (w/v), for example 1.800% (w/v);
(iii.e) benzalkonium chloride at an amount comprised from 0.005% (w/v) to 3% (w/v), preferably comprised from 0.01% (w/v) to 2% (w/v), more preferably comprised from 0.05% (w/v) to 1.5% (w/v), for example 0.100% (w/v);
(iii. f) water, preferably purified water or water for injections, up to a volume of 10 ml of said composition.

Furthermore, disclosed in the present description is a method for the curative treatment of an inflammation of the ocular adnexa or inflammation of the eyeball in a subject in need, wherein said treatment method comprises administering said composition to said subject.

In an embodiment, the administration of from 1 drop to 4 drops, from 1 to 3 times a day, of a composition from 0.1% to 0.5%, preferably 0.2% (0.2 g/100 ml).

### EXAMPLES

### Example 1: Preparation of compositions according to the present invention

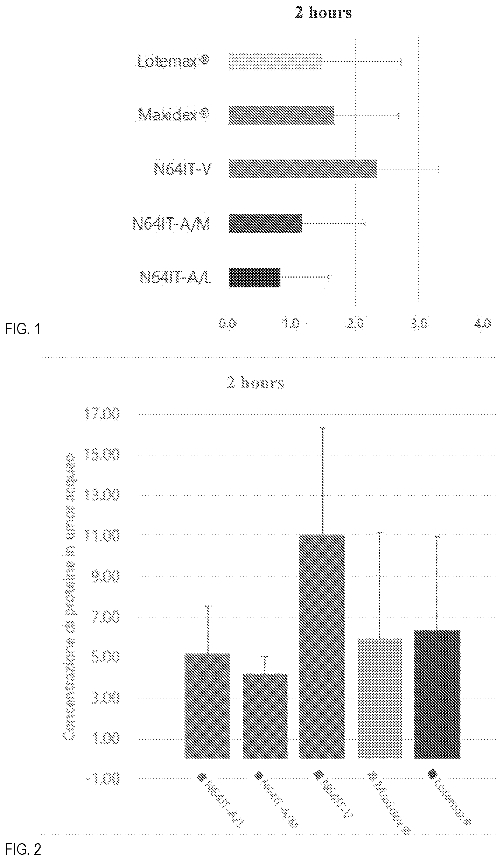

Two different liquid compositions subject of the present invention were prepared, each with a different dose of budesonide: 1) a first dose at 0.10% w/v (N64IT-A/L), and 2) a second high dose at 0.20% w/v (N64IT-A/M)(N64IT-A/H; the high dose did not appear in the current Figure 2, nor in the PGE2 diagram. For homogeneity we decided to omit the high dose data).

Such two liquid compositions have the qualitative and quantitative composition (expressed in % w/v) reported in Table 5A below.

**Table 5A**

| Component | Function | Composition | |
|---|---|---|---|
| | | N64IT-A/L (%) | N64IT-A/M (%) |
| Micronized budesonide | Active | 0.100 | 0.200 |
| Polysorbate 80 | Surfactant | 0.030 | 0.030 |
| Disodium-EDTA | Chelating agent | 0.010 | 0.010 |
| Sodium-CMC | Suspending/thickening agent | 0.160 | 0.160 |
| Mannitol | Isotonic agent | 1.800 | 1.800 |
| Benzalkonium chloride | Preservative | 0.010 | 0.010 |
| Purified water | Liquid solvent/carrier | q.s. up to10 ml | |

Therefore, the concentration of budesonide is the only variable component in said two compositions.

The storage conditions and the period of stability before/after opening of containers containing the compositions are respectively:
- before opening: room temperature at 20°C; 6 months;
- after opening: 4°C; 8 weeks.

Table 5B below reports pH, viscosity (measured at 20°C) and osmolality values of the placebo composition discussed hereinafter in Table 5B, and of the compositions N64IT-A/L and N64IT-A/M of Table 5A above.

**Table 5B**

| Composition | pH | Viscosity (cPs) | Osmolality (mOsm) |
|---|---|---|---|
| N64IT-V (placebo) | 6.6 | 15 | 270 |
| N64IT-A/L (low dose) | 6.7 | 15 | 271 |
| N64IT-A/M (medium dose) | 6.6 | 14 | 275 |
| N64IT-A/H (high dose) | 6.6 | 18 | 277 |

### Example 2: Tests on the efficacy of the compositions prepared in Example 1 on the animal

The first (N64IT-A/L) and the second (N64IT-A/M) liquid composition of budesonide of Example 1 were used for an ophthalmic efficacy test on the animal (rabbit) according to the following trial protocol.

The protocol chosen to induce eye inflammation in the animal and to evaluate the efficacy of the substances administered as anti-inflammatory provides for an initial administration 6 hours before the inflammatory event (tissue injury, at time t=0), and observations temporally after 30 minutes, 1 hour and 2 hours of the evolution of the inflammation symptoms, according to the scheme of Table 6 below.

**Table 6**

| Experimental scheme: 6 animals per time point | | | | | |
|---|---|---|---|---|---|
| Time | -6 hours | t=0 | +30 min | +1 hour | +2 hours |
| Induction: collection of 200 µl of aqueous humour | | X | | | |
| Maxidex^{®} (0.1% Dexamethasone) | X | | | | |
| Lotemax^{®} (0.5% Loteprednol) | X | | | | |
| N64IT-V (placebo) | X | | | | |
| N64IT-A/L (first dose) | X | | | | |
| N64IT-A/M (second dose) | X | | | | |
| Slit lamp eve examination | | | X | X | X |
| Protein concentration assay in aqueous humour | | | X | X | X |
| PGE2 concentration assay in aqueous humour | | | X | X | X |

The efficacy trial protocol provided for: pre-treatment with the two budesonide compositions at different doses, pre-treatments in other groups of subjects with a placebo (N64IT-V; liquid carrier) and pre-treatment with two market reference products based on different corticosteroids (Maxidex^{®} containing 0.1% dexamethasone; Lotemax^{®} containing 0.5% loteprednol), for eye use.

In the efficacy evaluation (30 minutes, 1 hour and 2 hours after the inflammatory event), three different types of observations were conducted on the 6 animals per treatment group, aimed at establishing decrease in the inflammatory condition induced by a standard paracentesis (incision) of the corneal tissue.

The first observations relate to possible material suspended in the aqueous humour deriving from the inflammatory process determined by means of a slit lamp, used in the ophthalmic field to evaluate the anatomical and functional condition of the eye. According to this observation, the lower the presence of suspended material (corpuscles) in aqueous humour, the lower the detected condition of inflammation.

The results of the first observations are reported in graphical form in the attached Figure 1, after 2 hours.

Only this data is reported for solely for explanatory purposes.

In these figures, the column diagrams relating to each pre-treatment were reported, where such diagrams report - on the abscissa - a mean inflammation index (parameter of integer value comprised from 0 to 4) relative to the only eye of the animal in which the inflammation was induced.

From Figure 1 it can be inferred that, surprisingly, the first dose of the budesonide liquid composition (0.10% w/v; N64IT-A/L composition) is the most effective by virtue of a smaller amount of suspended corpuscles (flares). This greater efficacy is manifested both in comparison with all the other substances (placebo, carrier or cortisone agents other than budesonide) with which the pre-treatment was carried out, and in comparison with the second dose (0.20% w/v; N64IT-A/M) of the composition comprising budesonide.

The latter consideration is particularly unexpected, since a low dose could be expected to reveal an equivalent but not greater efficacy with respect to a higher dose.

The data of a second and a third test typical of the inflammatory phenomena on amounts of proteins and prostaglandins PGE2 in aqueous humour, in which these amounts are both direct indices of the inflammatory conditions in a tissue, were therefore evaluated: the higher the concentration of proteins and of PGE2, the more inflammatory conditions are present in said tissue.

The results of the second observations relating to protein concentration are reported in graphical form in the attached Figure 2 after 2 hours. Since the PGE2 concentration values are in line with the protein values, a separate diagram relating to PGE2 at 2 hours is not reported in the attached figures.

In these figures, the column diagrams relating to each pre-treatment were reported, where such diagrams report - on the ordinate - a protein concentration (expressed in ng/ml, nanograms of proteins per millilitre of aqueous humour) relative to the only eye of the animal in which the inflammation was induced.

A comparison between the two doses of the compositions subject of the present invention shows a substantial equivalence of effectiveness.

A comparison between the compositions subject of the present invention and the other compositions tested once again shows a better and significant effectiveness of the compositions subject of the invention.

This better activity of the compositions subject of the present invention is particularly marked, especially after 2 hours of tissue injury.

Object of the present invention is a composition comprising a mixture comprising, or alternatively, consisting of:
(i) budesonide, or a salt thereof, or mixtures thereof, in form of micronized powder, wherein said micronizec powder comprises powder particles having an average size distribution comprised from 0.05 µm to 20 µm;
(ii) at least one suspending/thickening agent selected from the group comprising, or alternatively, consisting of: cellulose, carboxymethylcellulose (CMC), sodium carboxymethylcellulose (Na-CMC), refined carboxymethylcelluloses, or mixtures thereof, wherein said composition comprises only the water-soluble fractions of CMC, Na-CMC and/or refined carboxymethylcelluloses, wherein said water-soluble fractions of carboxymethylcellulose (CMC), sodium carboxymethylcellulose (Na-CMC) and/ or refined carboxymethylcelluloses exclude products containing or, alternatively, consisting of microcrystalline cellulose and sodium carboxymethylcellulose at variable ratios and having non-water-soluble fractions, and optionally
   (a.2) one or more physiologically and/or pharmacologically acceptable excipient, wherein said composition is for use in a method for the curative treatment of an inflammation of the ocular adnexa or an inflammation of the eyeball in a subject in need. Preferably, in said composition the amount of said (i) budesonide, or salt thereof, or mixtures thereof is comprised from 0.005% (w/v) to 3% (w/v) by weight with respect to a total volume of said composition, preferably comprised from 0.01% (w/v) to 2% (w/v), even more preferably comprised from 0.05% (w/v) to 1.5% (w/v), for example 0.1% (w/v), 0.2% (w/v) or 0.5% (w/v). Preferably, in said composition said refined carboxymethylcelluloses have a degree of substitution (DS), defined as the average number of hydroxyl groups substituted with anhydroglucose units in said refined CMCs, comprised from 0.65 to 0.90 or comprised from 0.80 to 0.95, preferably comprised from 0.80 to 0.95. Preferably, in said composition said one or more physiologically and/or pharmacologically acceptable excipients is selected from the group comprising or, alternatively, consisting of: a preservative, preferably potassium sorbate and/or benzalkonium chloride, a surfactant, preferably polyoxyethylene (20) sorbitan monooleate, a chelating agent, preferably ethylenediaminetetraacetic acid (EDTA), or a salt thereof, or mixtures thereof, an isotonic agent, preferably mannitol or sodium chloride, a carrier, preferably an aqueous carrier, more preferably water, or mixtures thereof. Preferably, the composition comprises:
   (i) budesonide, or a salt thereof, or mixtures thereof, as defined in appended claim 1;
   (ii) carboxymethylcellulose (CMC), sodium carboxymethylcellulose (Na-CMC), and/or refined carboxymethylcelluloses, preferably in cross-linked form/s, or mixtures thereof, as defined in appended claim 1;
   (iii.a) potassium sorbate;
   (iii.b) polyoxyethylene (20) sorbitan monooleate (Polysorbate 80);
   (iii.c) ethylenediaminetetraacetic acid (EDTA), or a salt thereof, or mixtures thereof;
   (iii.d) mannitol or sodium chloride;
   (iii.e) benzalkonium chloride;
   (iii.f) water, preferably purified water or water for injections;
   preferably, said composition being devoid of opaque ingredients, i.e., ingredients additional to said (i) budesonide, or salt thereof, or mixtures thereof suspended in said composition.

Preferably, the composition comprises:
(i) a budesonide, or a salt thereof, or mixtures thereof, as defined in appended claim 1;
(ii) carboxymethylcellulose (CMC), sodium carboxymethylcellulose (Na-CMC), and/or refined carboxymethylcelluloses, as defined in appended claim 1, preferably in cross-linked form/s, or mixtures thereof;
(iii.a) potassium sorbate;
(iii.b) polyoxyethylene (20) sorbitan monooleate (Polysorbate 80);
(iii.c) ethylenediaminetetraacetic acid (EDTA), or a salt thereof, or mixtures thereof;
(iii.d) mannitol or sodium chloride;
(iii.e) benzalkonium chloride;
(iii.f) water, preferably purified water or water for injections;
preferably, said composition being devoid of opaque ingredients, i.e., ingredients additional to said (i) budesonide, or salt thereof, or mixtures thereof suspended in said composition. Preferably, the composition comprises (amounts expressed as weight with respect to the total volume of said composition; w/v):
   (i) budesonide, or a salt thereof, or mixtures thereof comprised from 0.005% (w/v) to 3% (w/v), preferably comprised from 0.01% (w/v) to 2% (w/v), even more preferably comprised from 0.05% (w/v) to 1.5% (w/v), for example 0.1% (w/v), 0.2% (w/v) or 0.5% (w/v);
   (ii) cellulose, carboxymethylcellulose (CMC), sodium carboxymethylcellulose (Na-CMC), refined carboxymethylcelluloses preferably in cross-linked form/s, or mixtures thereof comprised from 0.005% (w/v) to 3% (w/v), preferably comprised from 0.01% (w/v) to 2% (w/v), more preferably comprised from 0.01% (w/v) to 2% (w/v) even more preferably comprised from 0.05% (w/v) to 1.5% (w/v), for example 0.160% (w/v);
   (iii.a) potassium sorbate comprised from 0.005% (w/v) to 3% (w/v), preferably comprised from 0.01% (w/v) to 2% (w/v), more preferably comprised from 0.05% (w/v) to 1.5% (w/v), for example 0.100% (w/v);
   (iii.b) polyoxyethylene (20) sorbitan monooleate (Polysorbate 80) comprised from 0.001% (w/v) to 2% (w/v), preferably comprised from 0.005% (w/v) to 1% (w/v), even more preferably comprised from 0.01% (w/v) to 1.5% (w/v), for example 0.030% (w/v);
   (iii.c) ethylenediaminetetraacetic acid (EDTA), or a salt thereof, or mixtures thereof comprised from 0.005% (w/v) to 3% (w/v), preferably comprised from 0.01% (w/v) to 2% (w/v), more preferably comprised from 0.05% (w/v) to 1.5% (w/v), for example 0.100% (w/v);
   (iii.d) mannitol or sodium chloride comprised from 0.01% (w/v) to 10% (w/v), preferably comprised from 0.1% (w/v) to 7% (w/v), more preferably comprised from 0.5% (w/v) to 5% (w/v), even more preferably comprised from 0.8% (w/v) to 3% (w/v), for example 1.800% (w/v);
   (iii.e) benzalkonium chloride comprised from 0.005% (w/v) to 3% (w/v), preferably comprised from 0.01% (w/v) to 2% (w/v), more preferably comprised from 0.05% (w/v) to 1.5% (w/v), for example 0.100% (w/v); (iii. f) water, preferably purified water or water for injections, up to a volume of 10 ml of said composition. Preferably, in the composition said micronized powder comprises powder particles having an average size distribution comprised from 0.1 µm to 10 µm, preferably comprised from 0.2 µm to 5 µm, more preferably comprised from 0.25 µm to 3.75 µm.

Preferably, said micronized powder comprises powder particles having an average size distribution comprised from 0.40 µm to 3.0 µm.

Preferably, the composition is a suspension in water, preferably wherein said suspension has a pH value (at 20°C and 1 Atm) comprised from 6±0.1 to 8±0.1, preferably comprised from 6.5±0.1 to 7.5±0.1, even more preferably from 6.7±0.1 to 7.3±0.1.

Preferably, the composition is for use in a method that comprises an administration of said composition through the ocular topical route, preferably on the ocular adnexa or on the eyeball, more preferably on the eyelid, conjunctiva, cornea and/or vitreous, even more preferably on the cornea (with the eye open) or on the eyelid (with the eye closed).

Preferably, said disorder or said ailment or said disease is an eye inflammation selected from the group comprising, or alternatively, consisting of allergic conjunctivitis, allergic keratitis, keratitis, dry eye syndrome, allergy eye redness, and swelling from ocular phlogosis.

## Claims

1. A composition comprising a mixture comprising, or alternatively, consisting of:
(i) budesonide, or a salt thereof, or mixtures thereof, in form of micronized powder, wherein said micronized powder comprises powder particles having an average size distribution comprised from 0.05 µm to 20 µm;
(ii) at least one suspending/thickening agent selected from the group comprising or, alternatively, consisting of: carboxymethylcellulose (CMC), sodium carboxymethylcellulose (Na-CMC) and refined carboxymethylcelluloses, or mixtures thereof, wherein said composition comprises only the water-soluble fractions of CMC, Na-CMC and/or refined carboxymethylcelluloses,
wherein said water-soluble fractions of carboxymethylcellulose (CMC), sodium carboxymethylcellulose (Na-CMC) and/or refined carboxymethylcelluloses exclude products containing or, alternatively, consisting of microcrystalline cellulose and sodium carboxymethylcellulose at variable ratios and having non-water-soluble fractions, and optionally
(iii) one or more physiologically and/or pharmacologically acceptable excipient,
wherein said composition is for use in a method for the curative treatment of an inflammation of the ocular adnexa or an inflammation of the eyeball in a subject in need.

2. Composition for use according to claim 1, wherein an amount of said (i) budesonide, or salt thereof, or mixtures thereof is comprised from 0.005% (w/v) to 3% (w/v) by weight with respect to a total volume of said composition, preferably comprised from 0.01% (w/v) to 2% (w/v), even more preferably comprised from 0.05% (w/v) to 1.5% (w/v), for example 0.1% (w/v), 0.2% (w/v) or 0.5% (w/v).

3. The composition for use according to any one of the preceding claims, wherein said refined carboxymethylcelluloses have a degree of substitution (DS), defined as the average number of hydroxyl groups substituted with anhydroglucose units in said refined CMCs, comprised from 0.65 to 0.90 or comprised from 0.80 to 0.95, preferably comprised from 0.80 to 0.95.

4. The composition for use according to any one of the preceding claims, wherein said one or more physiologically and/or pharmacologically acceptable excipients is selected from the group comprising or, alternatively, consisting of: a preservative, preferably potassium sorbate and/or benzalkonium chloride, a surfactant, preferably polyoxyethylene (20) sorbitan monooleate, a chelating agent, preferably ethylenediaminetetraacetic acid (EDTA), or a salt thereof, or mixtures thereof, an isotonic agent, preferably mannitol or sodium chloride, a carrier, preferably an aqueous carrier, more preferably water, or mixtures thereof.

5. Composition for use according to any one of the preceding claims, comprising:
(i) budesonide, or a salt thereof, or mixtures thereof; in form of micronized powder, wherein said micronized powder comprises powder particles having an average size distribution comprised from 0.05 µm to 20 µm;
(ii) carboxymethylcellulose (CMC), sodium carboxymethylcellulose (Na-CMC), and/or refined carboxymethylcelluloses, preferably in cross-linked form/s, or mixtures thereof;
(iii.a) potassium sorbate;
(iii.b) polyoxyethylene (20) sorbitan monooleate (Polysorbate 80);
(iii.c) ethylenediaminetetraacetic acid (EDTA), or a salt thereof, or mixtures thereof;
(iii.d) mannitol or sodium chloride;
(iii.e) benzalkonium chloride;
(iii.f) water, preferably purified water or water for injections;
preferably, said composition being devoid of opaque ingredients, i.e., ingredients additional to said (i) budesonide, or salt thereof, or mixtures thereof suspended in said composition.

6. The composition for use according to the preceding claim, comprising (amounts expressed as weight with respect to the total volume of said composition; w/v):
(i) budesonide, or a salt thereof, or mixtures thereof in form of micronized powder, wherein said micronized powder comprises powder particles having an average size distribution comprised from 0.05 µm to 20 µm, comprised from 0.005% (w/v) to 3% (w/v), preferably comprised from 0.01% (w/v) to 2% (w/v), even more preferably comprised from 0.05% (w/v) to 1.5% (w/v), for example 0.1% (w/v), 0.2% (w/v) or 0.5% (w/v);
(ii) carboxymethylcellulose (CMC), sodium carboxymethylcellulose (Na-CMC), refined carboxymethylcelluloses preferably in cross-linked form/s, or mixtures thereof comprised from 0.005% (w/v) to 3% (w/v), preferably comprised from 0.01% (w/v) to 2% (w/v), even more preferably comprised from 0.05% (w/v) to 1.5% (w/v), for example 0.160% (w/v);
(iii.a) potassium sorbate comprised from 0.005% (w/v) to 3% (w/v), preferably comprised from 0.01% (w/v) to 2% (w/v), more preferably comprised from 0.05% (w/v) to 1.5% (w/v), for example 0.100% (w/v);
(iii.b) polyoxyethylene (20) sorbitan monooleate (Polysorbate 80) comprised from 0.001% (w/v) to 2% (w/v), preferably comprised from 0.005% (w/v) to 1% (w/v), even more preferably comprised from 0.01% (w/v) to 1.5% (w/v), for example 0.030% (w/v);
(iii.c) ethylenediaminetetraacetic acid (EDTA), or a salt thereof, or mixtures thereof comprised from 0.005% (w/v) to 3% (w/v), preferably comprised from 0.01% (w/v) to 2% (w/v), more preferably comprised from 0.05% (w/v) to 1.5% (w/v), for example 0.100% (w/v);
(iii.d) mannitol or sodium chloride comprised from 0.01% (w/v) to 10% (w/v), preferably comprised from 0.1% (w/v) to 7% (w/v), more preferably comprised from 0.5% (w/v) to 5% (w/v), even more preferably comprised from 0.8% (w/v) to 3% (w/v), for example 1.800% (w/v);
(iii.e) benzalkonium chloride comprised from 0.005% (w/v) to 3% (w/v), preferably comprised from 0.01% (w/v) to 2% (w/v), more preferably comprised from 0.05% (w/v) to 1.5% (w/v), for example 0.100% (w/v); (iii.f) water, preferably purified water or water for injections, up to a volume of 10 ml of said composition.

7. The composition for use according to any one of the preceding claims, wherein said micronized powder comprises powder particles having an average size distribution comprised from 0.1 µm to 10 µm, preferably comprised from 0.2 µm to 5 µm, more preferably comprised from 0.25 µm to 3.75 µm.

8. The composition for use according to any one of the preceding claims, wherein said micronized powder comprises powder particles having an average size distribution comprised from 0.40 µm to 3.0 µm.

9. The composition for use according to any one of the preceding claims, wherein said composition is a suspension in water, preferably wherein said suspension has a pH value (at 20°C and 1 Atm) comprised from 6±0.1 to 8±0.1, preferably comprised from 6.5±0.1 to 7.5±0.1, even more preferably from 6.7±0.1 to 7.3±0.1.

10. The composition for use according to any one of the preceding claims, wherein said method comprises an administration of said composition through the ocular topical route, preferably on the ocular adnexa or on the eyeball, more preferably on the eyelid, conjunctiva, cornea and/or vitreous, even more preferably on the cornea (with eye open) or on the eyelid (with the eye closed).

11. The composition for use according to any one of the preceding claims, wherein said disorder or said ailment or said disease is an eye inflammation selected from the group comprising, or alternatively, consisting of allergic conjunctivitis, allergic keratitis, keratitis, dry eye syndrome, allergy eye redness, and swelling from ocular phlogosis.

## Patentansprüche

1. Zusammensetzung, umfassend eine Mischung, umfassend oder alternativ bestehend aus:
(i) Budesonid oder ein(em) Salz davon oder Mischungen davon in Form von mikronisiertem Pulver, wobei das mikronisierte Pulver Pulverpartikel mit einer durchschnittlichen Größenverteilung im Bereich von 0,05 µm bis 20 µm umfasst;
(ii) mindestens Suspendier-/Verdickungsmittel(n) ausgewählt aus der Gruppe umfassend oder alternativ bestehend aus: Carboxymethylcellulose (CMC), Natriumcarboxymethylcellulose (Na-CMC) und raffinierte(n) Carboxymethylcellulosen oder Mischungen davon, wobei die Zusammensetzung nur die wasserlöslichen Fraktionen von CMC, Na-CMC und/oder raffinierten Carboxymethylcellulosen umfasst, wobei die wasserlöslichen Fraktionen von Carboxymethylcellulose (CMC), Natriumcarboxymethylcellulose (Na-CMC) und/oder raffinierten Carboxymethylcellulosen Produkte ausschließen, die mikrokristalline Cellulose und Natriumcarboxymethylcellulose in variablen Verhältnissen enthalten oder alternativ daraus bestehen und nicht wasserlösliche Fraktionen aufweisen, und optional
(iii) einen(m) oder mehrere(n) physiologisch und/oder pharmakologisch verträgliche(n) Hilfsstoffe(n), wobei die Zusammensetzung zur Verwendung in einem Verfahren zur kurativen Behandlung einer Entzündung der Augenadnexen oder einer Entzündung des Augapfels bei einem bedürftigen Patienten bestimmt ist.

2. Zusammensetzung zur Verwendung nach Anspruch 1, wobei eine Menge des (i) Budesonids oder Salzes davon oder Mischungen davon im Bereich von 0,005 Gew.- % (w/v) bis 3 Gew.- % (w/v) in Bezug auf ein Gesamtvolumen der Zusammensetzung, vorzugsweise im Bereich von 0,01 Gew.- % (w/v) bis 2 Gew.- % (w/v), noch bevorzugter im Bereich von 0,05 Gew.- % (w/v) bis 1,5 Gew.- % (w/v), zum Beispiel 0,1 Gew.-% (w/v), 0,2 Gew.- % (w/v) oder 0,5 Gew.- % (w/v) liegt.

3. Zusammensetzung zur Verwendung nach einem der vorhergehenden Ansprüche, wobei die raffinierten Carboxymethylcellulosen einen Substitutionsgrad (DS) aufweisen, definiert als die durchschnittliche Anzahl von Hydroxylgruppen, die mit Anhydroglucoseeinheiten in den raffinierten CMCs substituiert sind, im Bereich von 0,65 bis 0,90 oder im Bereich von 0,80 bis 0,95, vorzugsweise im Bereich von 0,80 bis 0,95.

4. Zusammensetzung zur Verwendung nach einem der vorhergehenden Ansprüche, wobei der eine oder die mehreren physiologisch und/oder pharmakologisch verträglichen Hilfsstoffe ausgewählt ist/sind aus der Gruppe umfassend oder alternativ bestehend aus: Konservierungsmittel(n), vorzugsweise Kaliumsorbat und/oder Benzalkoniumchlorid, ein(em) Tensid, vorzugsweise Polyoxyethylen (20)-sorbitanmonooleat, einen(m) Chelatbildner, vorzugsweise Ethylendiamintetraessigsäure (EDTA), oder ein(em) Salz davon, oder Mischungen davon, isotonische(n) Mittel(n), vorzugsweise Mannit oder Natriumchlorid, einen(m) Träger, vorzugsweise einen(m) wässrigen Träger, bevorzugter Wasser, oder Mischungen davon.

5. Zusammensetzung zur Verwendung nach einem der vorhergehenden Ansprüche, umfassend:
(i) Budesonid oder ein Salz davon oder Mischungen davon; in Form von mikronisiertem Pulver, wobei das mikronisierte Pulver Pulverpartikel mit einer durchschnittlichen Größenverteilung im Bereich von 0,05 µm bis 20 µm umfasst;
(ii) Carboxymethylcellulose (CMC), Natriumcarboxymethylcellulose (Na-CMC) und/oder raffinierte Carboxymethylcellulosen, vorzugsweise in vernetzter Form/vernetzten Formen oder Mischungen davon;
(iii.a) Kaliumsorbat;
(iii.b) Polyoxyethylen (20)-sorbitanmonooleat (Polysorbat 80)
(iii.c) Ethylendiamintetraessigsäure (EDTA) oder ein Salz davon oder Mischungen davon;
(iii.d) Mannit oder Natriumchlorid;
(iii.e) Benzalkoniumchlorid;
(iii.f) Wasser, vorzugsweise gereinigtes Wasser oder Wasser für Injektionszwecke;
vorzugsweise wobei die Zusammensetzung frei von opaken Bestandteilen ist, d. h. Bestandteilen, die zusätzlich zu dem (i) Budesonid oder Salz davon oder Mischungen davon in der Zusammensetzung suspendiert sind.

6. Zusammensetzung zur Verwendung nach dem vorhergehenden Anspruch, umfassend (Mengen ausgedrückt als Gewicht in Bezug auf das Gesamtvolumen der Zusammensetzung; w/v):
(i) Budesonid oder ein Salz davon oder Mischungen davon in Form von mikronisiertem Pulver, wobei das mikronisierte Pulver Pulverpartikel mit einer durchschnittlichen Größenverteilung im Bereich von 0,05 µm bis 20 µm, im Bereich von 0,005 % (w/v) bis 3 % (w/v), vorzugsweise im Bereich von 0,01 % (w/v) bis 2 % (w/v), noch bevorzugter im Bereich von 0,05 % (w/v) bis 1,5 % (w/v), zum Beispiel 0,1 % (w/v), 0,2 % (w/v) oder 0,5 % (w/v) umfasst;
(i) Carboxymethylcellulose (CMC), Natriumcarboxymethylcellulose (Na-CMC), raffinierte Carboxymethylcellulosen, vorzugsweise in vernetzter Form/vernetzten Formen, oder Mischungen davon, im Bereich von 0,005 % (w/v) bis 3 % (w/v), vorzugsweise im Bereich von 0,01 % (w/v) bis 2 % (w/v), noch bevorzugter im Bereich von 0,05 % (w/v) bis 1,5 % (w/v), zum Beispiel 0,160 % (w/v);
(iii.a) Kaliumsorbat im Bereich von 0,005 % (w/v) bis 3 % (w/v), vorzugsweise im Bereich von 0,01 % (w/v) bis 2 % (w/v), bevorzugter im Bereich von 0,05 % (w/v) bis 1,5 % (w/v), zum Beispiel 0,100 % (w/v);
(iii.b) Polyoxyethylen (20)-sorbitanmonooleat (Polysorbat 80), im Bereich von 0,001 % (w/v) bis 2 % (w/v), vorzugsweise im Bereich von 0,005 % (w/v) bis 1 % (w/v), noch bevorzugter im Bereich von 0,01 % (w/v) bis 1,5 % (w/v), zum Beispiel 0,030 % (w/v);
(iii.c) Ethylendiamintetraessigsäure (EDTA) oder ein Salz davon oder Mischungen davon, im Bereich von 0,005 % (w/v) bis 3 % (w/v), vorzugsweise im Bereich von 0,01 % (w/v) bis 2 % (w/v), noch bevorzugter im Bereich von 0,05 % (w/v) bis 1,5 % (w/v), zum Beispiel 0,100 % (w/v);
(iii.d) Mannitol oder Natriumchlorid, im Bereich von 0,01 % (w/v) bis 10 % (w/v), vorzugsweise im Bereich von 0,1 % (w/v) bis 7 % (w/v), bevorzugter im Bereich von 0,5 % (w/v) bis 5 % (w/v), noch bevorzugter im Bereich von 0,8 % (w/v) bis 3 % (w/v), zum Beispiel 1.800 % (w/v);
(iii.e) Benzalkoniumchlorid, im Bereich von 0,005 % (w/v) bis 3 % (w/v), vorzugsweise im Bereich von 0,01 % (w/v) bis 2 % (w/v), noch bevorzugter im Bereich von 0,05 % (w/v) bis 1,5 % (w/v), zum Beispiel 0,100 % (w/v);
(iii.f) Wasser, vorzugsweise gereinigtes Wasser oder Wasser für Injektionszwecke, bis zu einem Volumen von 10 ml der Zusammensetzung.

7. Zusammensetzung zur Verwendung nach einem der vorhergehenden Ansprüche, wobei das mikronisierte Pulver Pulverpartikel mit einer durchschnittlichen Größenverteilung im Bereich von 0,1 µm bis 10 µm, vorzugsweise im Bereich von 0,2 µm bis 5 µm, bevorzugter im Bereich von 0,25 µm bis 3,75 µm umfasst.

8. Zusammensetzung zur Verwendung nach einem der vorhergehenden Ansprüche, wobei das mikronisierte Pulver Pulverpartikel mit einer durchschnittlichen Größenverteilung im Bereich von 0,40 µm bis 3,0 µm umfasst.

9. Zusammensetzung zur Verwendung nach einem der vorhergehenden Ansprüche, wobei die Zusammensetzung eine Suspension in Wasser ist, wobei die Suspension vorzugsweise einen pH-Wert (bei 20 °C und 1 Atm) im Bereich von 6±0,1 bis 8±0,1, vorzugsweise im Bereich von 6,5±0,1 bis 7,5±0,1, noch bevorzugter von 6,7±0,1 bis 7,3±0,1 aufweist.

10. Zusammensetzung zur Verwendung nach einem der vorhergehenden Ansprüche, wobei das Verfahren eine Verabreichung der Zusammensetzung auf dem topischen Weg des Auges umfasst, vorzugsweise auf den Augenadnexen oder auf dem Augapfel, bevorzugter auf dem Augenlid, der Bindehaut, der Hornhaut und/oder dem Glaskörper, noch bevorzugter auf der Hornhaut (bei geöffnetem Auge) oder auf dem Augenlid (bei geschlossenem Auge).

11. Zusammensetzung zur Verwendung nach einem der vorhergehenden Ansprüche, wobei die Erkrankung oder das Leiden oder die Krankheit eine Augenentzündung ist, ausgewählt aus der Gruppe umfassend oder alternativ bestehend aus allergische(r) Konjunktivitis, allergische(r) Keratitis, Keratitis, Syndrom des trockenen Auges, allergische(r) Augenrötung und Schwellung von Augenphlogose.

## Revendications

1. Composition comprenant un mélange comprenant, ou alternativement, constitué :
(i) de budésonide, ou un de ses sels, ou leurs mélanges, sous forme de poudre micronisée, dans laquelle ladite poudre micronisée comprend des particules de poudre ayant une distribution de taille moyenne comprise entre 0,05 µm et 20 µm ;
(ii) d'au moins un agent de suspension/épaississant choisi dans le groupe comprenant ou, alternativement, constitué : de la carboxyméthylcellulose (CMC), du carboxyméthylcellulose sodique (Na-CMC) et des carboxyméthylcelluloses raffinées, ou leurs mélanges, dans laquelle ladite composition ne comprend que les fractions hydrosolubles de la CMC, du Na-CMC et/ou des carboxyméthylcelluloses raffinées,
dans laquelle lesdites fractions hydrosolubles de la carboxyméthylcellulose (CMC), du carboxyméthylcellulose sodique (Na-CMC) et/ou des carboxyméthylcelluloses raffinées excluent les produits contenant ou, alternativement, constitués de cellulose microcristalline et de carboxyméthylcellulose sodique à des ratios variables et ayant des fractions non solubles dans l'eau, et éventuellement
(iii) d'un ou plusieurs excipients physiologiquement et/ou pharmacologiquement acceptables,
dans laquelle ladite composition est destinée à être utilisée dans une méthode de traitement curatif d'une inflammation des annexes de l'œil ou d'une inflammation du globe oculaire chez un sujet qui en a besoin.

2. Composition destinée à être utilisée selon la revendication 1, dans laquelle une quantité dudit (i) budésonide, ou de son sel, ou de leurs mélanges est comprise entre 0,005 % (p/v) et 3 % (p/v) en poids par rapport au volume total de ladite composition, de préférence comprise entre 0,01 % (p/v) et 2 % (p/v), plus préférablement comprise entre 0,05 % (p/v) et 1,5 % (p/v), par exemple 0,1 % (p/v), 0,2 % (p/v) ou 0,5 % (p/v).

3. Composition destinée à être utilisée selon l'une quelconque des revendications précédentes, dans laquelle lesdites carboxyméthylcelluloses raffinées ont un degré de substitution (DS), défini comme le nombre moyen de groupes hydroxyle substitués par des unités d'anhydroglucose dans lesdites CMC raffinées, compris entre 0,65 et 0,90 ou compris entre 0,80 et 0,95, de préférence compris entre 0,80 et 0,95.

4. Composition destinée à être utilisée selon l'une quelconque des revendications précédentes, dans laquelle lesdits un ou plusieurs excipients physiologiquement et/ou pharmacologiquement acceptables sont choisis dans le groupe comprenant ou, alternativement, constitué de : un conservateur, de préférence le sorbate de potassium et/ou le chlorure de benzalkonium, un tensioactif, de préférence le monooléate de polyoxyéthylène (20) sorbitane, un agent chélatant, de préférence l'acide éthylènediaminetétraacétique (EDTA), ou un de ses sels, ou leurs mélanges, un agent isotonique, de préférence le mannitol ou le chlorure de sodium, un support, de préférence un support aqueux, plus préférablement de l'eau, ou leurs mélanges.

5. Composition destinée à être utilisée selon l'une quelconque des revendications précédentes, comprenant :
(i) du budésonide, ou un de ses sels, ou leurs mélanges ; sous forme de poudre micronisée, dans laquelle ladite poudre micronisée comprend des particules de poudre ayant une distribution de taille moyenne comprise entre 0,05 µm et 20 µm ;
(ii) de la carboxyméthylcellulose (CMC), du carboxyméthylcellulose sodique (Na-CMC), et/ou des carboxyméthylcelluloses raffinées, de préférence sous forme réticulée/des formes réticulées, ou leurs mélanges ;
(iii.a) du sorbate de potassium ;
(iii.b) du monooléate de polyoxyéthylène (20) sorbitane (Polysorbate 80) ;
(iii.c) de l'acide éthylènediaminetétraacétique (EDTA), ou un de ses sels, ou leurs mélanges ;
(iii.d) du mannitol ou du chlorure de sodium ;
(iii.e) du chlorure de benzalkonium ;
(iii.f) de l'eau, de préférence de l'eau purifiée ou de l'eau pour injections ;
de préférence, ladite composition étant dépourvue d'ingrédients opaques, c'est-à-dire, d'ingrédients additionnels audit (i) budésonide, ou à son sel, ou à leurs mélanges en suspension dans ladite composition.

6. Composition destinée à être utilisée selon la revendication précédente, comprenant (quantités exprimées en poids par rapport au volume total de ladite composition ; p/v) :
(i) du budésonide, ou un de ses sels, ou leurs mélanges sous forme de poudre micronisée, dans laquelle ladite poudre micronisée comprend des particules de poudre ayant une distribution de taille moyenne comprise entre 0,05 µm et 20 µm, comprise entre 0,005 % (p/v) et 3 % (p/v), de préférence comprise entre 0,01 % (p/v) et 2 % (p/v), plus préférablement comprise entre 0,05 % (p/v) et 1,5 % (p/v), par exemple 0,1 % (p/v), 0,2 % (p/v) ou 0,5 % (p/v) ;
(ii) de la carboxyméthylcellulose (CMC), du carboxyméthylcellulose sodique (Na-CMC), des carboxyméthylcelluloses raffinées, de préférence sous forme réticulée/des formes réticulées, ou leurs mélanges, compris entre 0,005 % (p/v) et 3 % (p/v), de préférence compris entre 0,01 % (p/v) et 2 % (p/v), plus préférablement encore compris entre 0,05 % (p/v) et 1,5 % (p/v), par exemple 0,160 % (p/v) ;
(iii.a) du sorbate de potassium compris entre 0,005 % (p/v) et 3 % (p/v), de préférence compris entre 0,01 % (p/v) et 2 % (p/v), plus préférablement compris entre 0,05 % (p/v) et 1,5 % (p/v), par exemple 0,100 % (p/v) ;
(iii. b) du monooléate de polyoxyéthylène (20) sorbitane (Polysorbate 80) compris entre 0,001 % (p/v) et 2 % (p/v), de préférence compris entre 0,005 % (p/v) et 1 % (p/v), plus préférablement encore compris entre 0,01 % (p/v) et 1,5 % (p/v), par exemple 0,030 % (p/v) ;
(iii.c) de l'acide éthylènediaminetétraacétique (EDTA), ou un de ses sels, ou leurs mélanges, compris entre 0,005 % (p/v) et 3 % (p/v), de préférence entre 0,01 % (p/v) et 2 % (p/v), plus préférablement compris entre 0,05 % (p/v) et 1,5 % (p/v), par exemple 0,100 % (p/v) ;
(iii.d) du mannitol ou du chlorure de sodium compris entre 0,01 % (p/v) et 10 % (p/v), de préférence compris entre 0,1 % (p/v) et 7 % (p/v), plus préférablement compris entre 0,5 % (p/v) et 5 % (p/v), plus préférablement encore compris entre 0,8 % (p/v) et 3 % (p/v), par exemple 1,800 % (p/v) ;
(iii.e) du chlorure de benzalkonium compris entre 0,005 % (p/v) et 3 % (p/v), de préférence compris entre 0,01 % (p/v) et 2 % (p/v), plus préférablement compris entre 0,05 % (p/v) et 1,5 % (p/v), par exemple 0,100 % (p/v) ;
(iii.f) de l'eau, de préférence de l'eau purifiée ou de l'eau pour injections, jusqu'à un volume de 10 ml de ladite composition.

7. Composition destinée à être utilisée selon l'une quelconque des revendications précédentes, dans laquelle ladite poudre micronisée comprend des particules de poudre ayant une distribution de taille moyenne comprise entre 0,1 µm et 10 µm, de préférence comprise entre 0,2 µm et 5 µm, plus préférablement comprise entre 0,25 µm et 3,75 µm.

8. Composition destinée à être utilisée selon l'une quelconque des revendications précédentes, dans laquelle ladite poudre micronisée comprend des particules de poudre ayant une distribution de taille moyenne comprise entre 0,40 µm et 3,0 µm.

9. Composition destinée à être utilisée selon l'une quelconque des revendications précédentes, dans laquelle ladite composition est une suspension dans l'eau, de préférence dans laquelle ladite suspension a une valeur de pH (à 20°C et 1 Atm) comprise entre 6 ± 0,1 et 8 ± 0,1, de préférence comprise entre 6,5 ± 0,1 et 7,5 ± 0,1, plus préférablement encore entre 6,7 ± 0,1 et 7,3 ± 0,1.

10. Composition destinée à être utilisée selon l'une quelconque des revendications précédentes, dans laquelle ladite méthode comprend une administration de ladite composition par voie topique oculaire, de préférence sur les annexes de l'œil ou sur le globe oculaire, plus préférablement sur la paupière, la conjonctive, la cornée et/ou le vitré, plus préférablement encore sur la cornée (avec l'œil ouvert) ou sur la paupière (avec l'œil fermé).

11. Composition destinée à être utilisée selon l'une quelconque des revendications précédentes, dans laquelle ledit trouble ou ladite affection ou ladite maladie est une inflammation de l'œil choisie dans le groupe comprenant, ou alternativement, constitué de la conjonctivite allergique, la kératite allergique, la kératite, le syndrome de l'œil sec, la rougeur des yeux due à une allergie et du gonflement dû à une phlogose de l'œil.
